(19) Europäisches Patentamt
European Patent Office
Office européen des brevets

(11) **EP 3 599 230 B1**

(12) **EUROPÄISCHE PATENTSCHRIFT**

(45) Veröffentlichungstag und Bekanntmachung des Hinweises auf die Patenterteilung:
**15.06.2022 Patentblatt 2022/24**

(21) Anmeldenummer: **19187984.0**

(22) Anmeldetag: **24.07.2019**

(51) Internationale Patentklassifikation (IPC):
**C07C 2/10** (2006.01)   **C07C 11/02** (2006.01)
**B01J 23/755** (2006.01)

(52) Gemeinsame Patentklassifikation (CPC):
**C07C 2/10; B01J 21/12; B01J 23/755; B01J 35/1042; B01J 35/1061; B01J 37/0201; B01J 37/08; B01J 37/088;** C07C 2523/755

(54) **VERFAHREN ZUR OLIGOMERISIERUNG VON BUTEN UNTER BESTIMMUNG DES ANTEILS AN SAURER KATALYSE**

METHOD FOR THE OLIGOMERIZATION OF BUTENE BY DETECTING THE PROPORTION OF ACID CATALYSIS

PROCÉDÉ D'OLIGOMÉRISATION DES BUTÈNES PAR DÉTERMINATION DU CONTENU D'UNE CATALYSE ACIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.07.2018 EP 18185533**

(43) Veröffentlichungstag der Anmeldung:
**29.01.2020 Patentblatt 2020/05**

(73) Patentinhaber: **Evonik Operations GmbH
45128 Essen (DE)**

(72) Erfinder:
• **Nadolny, Fabian
59821 Arnsberg (DE)**
• **Peitz, Stephan
45739 Oer-Erkenschwick (DE)**
• **Stochniol, Guido
45721 Haltern am See (DE)**
• **Franke, Robert
45772 Marl (DE)**
• **Alscher, Felix
01187 Dresden (DE)**
• **Breitkopf, Cornelia
01097 Dresden (DE)**
• **Reschetilowski, Wladimir
01445 Radebeul (DE)**

(74) Vertreter: **Evonik Patent Association
c/o Evonik Industries AG
IP Management
Bau 1042A/PB 15
Paul-Baumann-Straße 1
45772 Marl (DE)**

(56) Entgegenhaltungen:
**WO-A1-2006/111415**

• **HENTSCHEL ET AL.: "Untersuchungen an Nickeloxidmischkatalysatoren", CHEMIE TECHNIK, Bd. 34, Nr. 6, 1. Juni 1982 (1982-06-01), Seiten 313-316, XP002787656,**
• **WENDT G ET AL: "Structural and Catalytic Properties of NiO-Al2O3/SiO2 Catalysts for the Dimerization and Isomerization of Olefins", CATALYSIS 1987 : SAN DIEGO, CA, MAY 17-22, 1987 NORTH AMERICAN MEETING OF THE CATALYSIS SOCIETY ; 10 (SAN DIEGO/CALIF.) : 1987.05.17-22; [STUDIES IN SURFACE SCIENCE AND CATALYSIS; ISSN 0167-2991], ELSEVIER BV, NL, vol. 7, no. Part B, 1 January 1981 (1981-01-01), pages 978-992, XP009509239, ISSN: 0167-2991, DOI: 10.1016/S0167-2991(08)64708-4 [retrieved on 2009-01-07]**
• **HENTSCHEL ET AL: "Studies on nickel oxide mixed catalysts. Part IX", CHEMISCHE TECHNIK: TECHNISCHE CHEMIE - VERFAHRENSTECHNIK - UMWELTTECHNIK, DEUTSCHER VERLAG FUER GRUNDSTOFFINDUSTRIE GMBH, DE, vol. 34, 1 January 1982 (1982-01-01), pages 313-316, XP009509238, ISSN: 0045-6519**

**Beschreibung**

Die vorliegende Erfindung wie in den Ansprüchen definiert betrifft ein Verfahren zur Oligomerisierung von n-Butenen unter Verwendung eines Nickel-enthaltenden Alumosilikatkatalysators, wobei ein Produktgemisch erzeugt wird, bei dem das Verhältnis von 4,4-Dimethylhexen zu 3,4-Dimethylhexen bestimmt und überwacht wird.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit vier Kohlenstoffatomen (Buten) ein Olefin mit acht Kohlenstoffatomen (Okten) aufbauen. Die Oligomerisierung von zwei Molekülen miteinander wird auch Dimerisierung genannt. Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Bei den heterogen katalysierten Verfahren ist die Oligomerisierung an sauren Oligomerisierungskatalysatoren lange bekannt. Technisch werden saure Katalysatoren, z. B. Zeolithe oder Phosphorsäure, auf einem Support eingesetzt. Hierbei werden Isomerengemische von mehr oder weniger verzweigten Olefinen erhalten. Die Bezeichnung saure Katalyse oder saure Katalysatoren beschreibt hierbei eine Brønsted-Acidität, der Katalysator stellt also katalytische aktive Protonen bereit. Für die nicht-saure, heterogen katalysierte Oligomerisierung von Olefinen mit hoher Dimerenselektivität werden in der Technik häufig Nickelverbindungen auf Supportmaterialien eingesetzt, wobei das Nickel keine Protonen bereitstellt, sondern es eher als Elektronenpaarakzeptor wirkt (Lewis-Säure). So wird in der WO 95/14647 A1 ein Nickelkatalysator mit einem Supportmaterial, das aus den Komponenten Titanoxid und/oder Zirkoniumoxid, Siliciumdioxid und gegebenenfalls Aluminiumoxid besteht, für die Olefinoligomerisierung beschrieben. An diesen Katalysatoren werden Gemische linearer Butene in einer Selektivität von unter 75 % zu C8-Olefinen oligomerisiert. Dabei wird vermutet, dass die katalytische Aktivität von nickelbasierten, heterogenen Katalysatoren zur Oligomerisierung von Olefinen auf der Wechselwirkung zwischen Nickel-Kationen und Oberflächen-Aluminiumatomen basiert.

Bei der Oligomerisierung gibt es verschiedene Mechanismen, über die die Oligomerisierung ablaufen kann. Dazu gehört die saure Katalyse, bei der die Olefine mit den Säurezentrum eines Katalysators ein Carbenium-Ion bilden, welches mit der Doppelbindung eines weiteren Olefins reagieren kann und so eine neue C-C-Bindung bildet. Da das Carbenium-Ion an der höchst verzweigten Stelle des Kations am besten stabilisiert wird, werden stark verzweigte Oligomere gebildet, die nahezu ausschließlich für die Produktion von Kraftstoffen von Relevanz sind. Für die Weiterverarbeitung zu chemischen Endprodukten wie Weichmachern oder Tensiden werden technisch insbesondere Oligomere mit einer höheren Linearität benötigt. Ein weiterer Mechanismus ist der koordinative Mechanismus, bei dem das erste Olefin koordinativ an den Katalysator bindet. Dort kann sich ein weiteres Olefin anlagern und zur Ausbildung einer neuen C-C-Bindung und damit zur Bildung eines Oligomers führen. Die Produkte bei diesem Mechanismus sind typischerweise weniger stark verzweigt.

Die WO 2006/111415 A1 offenbart ein Verfahren zur Oligomerisierung von Olefinen mit 2 bis 6 Kohlenstoffatomen, bei dem der Austrag aus der Oligomerisierung in zwei Teilströme aufgetrennt wird. Ein Teilstrom wird dann einer Produktabtrennung unterworfen, um die gebildeten Oligomere abzutrennen und der andere Teilstrom wird zur Oligomerisierung zurückgeführt. Es wird keine Überwachung bestimmter Parameter offenbart.

Hentschel, et al. (Hentschel, et. Al "Untersuchungen an Nickeloxidmischkatalysatoren", Chemie Technik, Bd. 34, Nr. 6 (1982), S. 313-316) offenbart ein Verfahren zur Oligomerisierung von Buten. Die Untersuchungen wurden dabei an verschiedenen Nickel enthaltenden Alumosilikaten, die als Katalysatoren eingesetzt worden sind, durchgeführt. Es wird keine Überwachung bestimmter Parameter offenbart.

Im Vergleich zu bekannten Oligomerisierungsverfahren besteht die fortwährende Aufgabe neue Verfahrensansätze zu entwickeln, die zu einer Verbesserung des Umsatzes und/oder der Selektivität beim Einsatz in der Oligomerisierung von Olefinen hin zu lineareren Produkten führen. Die Aufgabe der vorliegenden Erfindung war deshalb ein Oligomerisierungsverfahren bereitzustellen, mit dem sich bei der Oligomerisierung höhere Selektivitäten und höhere Umsätze zu verstärkt linearen Produkten erzielen lassen, wobei dies anhand bestimmter Produktisomere überwacht wird.

Weiterhin besteht die Aufgabe, die Absättigung der Katalysator-Säurezentren mit Nickel quantifizieren zu können, um eine bessere Vorhersage der Eignung eines Katalysators für die Oligomerisierung in großtechnischen Mengen leisten zu können. Des Weiteren ist es Aufgabe der vorliegenden Erfindung, anhand der Interpretation der katalytischen Daten, Formierungsprozesse des Katalysators und die Desaktivierung spezifischer katalytischer Zentren während der Reaktion identifizieren zu können. Da die Oligomerisierung im kontinuierlichen Betrieb unter erhöhtem Druck durchgeführt wird, ist die Entnahme von Katalysatorproben während des Betriebs schwierig bis unmöglich. Für eine Abschätzung der weiteren Laufzeit sowie Beurteilung des zu erwartenden Produktspektrums ist es deshalb von großer Wichtigkeit, anhand der gebildeten Produkte den Zustand des Katalysators beurteilen zu können.

Die zugrundeliegende Aufgabe der vorliegenden Erfindung konnte mit dem Verfahren zur Oligomerisierung gemäß Anspruch 1 gelöst werden. Bevorzugte Ausführungsformen sind in den Unteransprüchen angegeben.

Das erfindungsgemäße Verfahren ist ein Verfahren zur Oligomerisierung von n-Butenen unter Verwendung eines mesoporösen, Nickel-enthaltenden Alumosilikatkatalysators, über den ein Eduktstrom, der die n-Butene enthält, unter Bildung eines Produktgemisches geleitet wird, dadurch gekennzeichnet, dass das Verhältnis von der Menge des gebildeten 4,4-Dimethylhexens zur Menge des gebildeten 3,4-Dimethylhexens in dem Produktgemisch überwacht und bei Überschreiten eines Grenzwerts für das Verhältnis (Menge 4,4-Dimethylhexen / Menge 3,4-Dimethylhexen) der Katalysator ausgetauscht wird, wobei der Grenzwert für das Verhältnis (Menge 4,4-Dimethylhexen / Menge 3,4-Dimethylhexen) maximal 0,05, vorzugsweise maximal 0,01, besonders bevorzugt maximal 0,005 beträgt.

Zur Bestimmung des Verhältnisses werden zunächst die Mengen der einzelnen Isomere vorzugsweise gaschromatographisch bestimmt und daraus das Verhältnis ermittelt. Um eine bessere Trennwirkung zu erzielen, kann die zu untersuchende Probe (Produktgemisch) vor dem Auftreffen auf die Trennsäule im Liner mit Wasserstoff als Trägergas über einem heterogenen Pdhaltigen Katalysator hydriert werden. Die daraus erhaltenen Alkane können besser unterschieden werden als die bei der Oligomerisierung gebildeten C8-Olefinisomere.

Das Verhältnis (Menge 4,4-Dimethylhexen / Menge 3,4-Dimethylhexen) kann kontinuierlich, d. h. durchgehend während des laufenden Verfahrens, oder diskontuinierlich, d. h. durch regelmäßige Entnahme einer Probe des Produktgemisches aus dem Verfahren während des Betriebs, bestimmt werden. Bevorzugt wird das Verhältnis (Menge 4,4-Dimethylhexen / Menge 3,4-Dimethylhexen) diskontinuierlich durch eine in regelmäßigen Abständen erfolgende Probenentnahme aus dem Produktgemisch bestimmt. Die Abstände zwischen den regelmäßigen Probenentnahmen können dabei frei gewählt werden und hängen von der betriebenen Anlage ab. Die Abstände zwischen den Probenentnahmen bei der diskontinuierlichen Bestimmung des Verhältnisses können grundsätzlich in Abständen von 1 bis 59 Minuten, 1 bis 23 Stunden, 1 bis 6 Tagen oder 1 bis 20 Wochen erfolgen. Möglich ist auch, dass die Abstände variieren, d. h. beispielsweise nach Einbau eines frischen Katalysators länger sind und mit der Zeit kürzer werden.

Es hat sich überraschend gezeigt, dass sich durch die Überwachung des Verhältnisses von 4,4-Dimethylhexen zu 3,4-Dimethylhexen besonders gute Produktqualitäten, sowie ein höherer Umsatz und/oder eine höhere Selektivität hin zu linearen Produkten, beim Einsatz im erfindungsgemäßen Oligomerisierungsverfahren erzielen lassen. Je kleiner dieses Verhältnis wird, desto geringer ist der Anteil an saurer Katalyse bei der Oligomerisierung und desto kleiner ist damit die Menge an entstandenen stärker verzweigten Oligomere. Kommt es jedoch zu einem Anstieg des Verhältnisses von 4,4-Dimethylhexen zu 3,4-Dimethylhexen, hat eine Formierung auf der Katalysatoroberfläche stattgefunden. Dadurch lässt sich für den weiteren Betrieb beurteilen, wie stark der mittlere Verzweigungsgrad der gebildeten Oligomere sein wird. Wird während des Verfahrens ein bestimmter Grenzwert überschritten, muss. der Katalysator ausgetauscht werden. Es ist dadurch möglich nahezu durchgehend eine hohe Linearität der gebildeten Oligomere zu erreichen, weil das Verfahren durch die Einstellung eines geeigneten Grenzwertes für das Verhältnis frühzeitig unterbrochen und der Katalysator ausgetauscht werden kann, also bevor vergleichsweise große Mengen an verzweigten Nebenprodukten überhaupt gebildet werden.

Ist durch Überschreiten des Grenzwertes ein Katalysatoraustausch erforderlich, wird der gebrauchte Katalysator durch einen frischen Katalysator ersetzt. Dies geschieht je nach Aufbau der Anlage in einer dem Fachmann bekannten Weise. Der frische Katalysator kann ein neu hergestellter Katalysator oder ein gebrauchter, aber regenerierter Katalysator sein.

Der Eduktstrom, der die n-Butene enthält, kann zwar auch ein Strom aus reinem n-Buten sein, was technisch aber kaum zu realisieren ist. Technische Gemische, die n-Butene enthalten und als Edukstrom eingesetzt werden können, sind Leichtbenzinfraktionen aus Raffinerien, $C_4$-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Beispielsweise können für das erfindungsgemäße Verfahren geeignete Gemische n-Butene aus der $C_4$-Fraktion eines Steamcrackers gewonnen werden. Dabei wird im ersten Schritt Butadien entfernt. Dies geschieht entweder durch Extraktion oder Extraktionsdestillation des Butadiens oder dessen Selektivhydrierung. In beiden Fällen wird ein praktisch butadienfreier $C_4$-Schnitt erhalten, das Raffinat I. Im zweiten Schritt wird Isobuten aus dem $C_4$-Strom entfernt, beispielsweise durch die Herstellung von Methyl-tert.-butylether (MTBE). Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt praktisch isobutenfreie $C_4$-Schnitt, das Raffinat II, enthält wie gewünscht die n-Butene und gegebenenfalls Butane.

In einer bevorzugten Ausführungsform werden das Raffinat I (butadienfreier C4-Schnitt des Steamcrackers) oder das Raffinat II (butadien- und isobutenfreier C4-Schnitt des Steamcrackers) dem Verfahren als Eduktstrom zugeführt.

Eine weitere Möglichkeit ein geeignetes Olefingemisch herzustellen, besteht darin, Raffinat I, Raffinat II oder ein ähnlich zusammengesetztes Kohlenwasserstoffgemisch in einer Reaktivkolonne zu hydroisomerisieren. Dabei kann u. a. ein Gemisch gewonnen werden, das aus 2-Butenen, geringen Anteilen 1-Buten und gegebenenfalls n-Butan sowie Isobutan und Isobuten besteht.

Je nach Ursprung und Aufarbeitung des Eduktstroms, können Verbindungen mit Heteroatomen, insbesondere stickstoff-, schwefel- und/oder sauerstoffhaltige Verbindungen, im Strom enthalten sein.

Das erfindungsgemäße Oligomerisierungsverfahren erfolgt vorzugsweise bei einer Temperatur im Bereich von 50 bis 200 °C, vorzugsweise im Bereich 60 bis 180 °C, besonders bevorzugt im Bereich von 60 bis 130°C. Der Druck beim erfindungsgemäßen Verfahren liegt vorzugsweise im Bereich von 10 bis 70 bar, besonders bevorzugt im Bereich von 15 bis 42 bar.

In einer weiteren bevorzugten Ausführungsform liegen die Edukte bei dem erfindungsgemäßen Verfahren in der flüssigen Phase vor. Sofern die Oligomerisierung in flüssiger Phase erfolgen soll, müssen die Parameter Druck und Temperatur hierfür so eingestellt werden, dass die Edukte in flüssiger Phase vorliegen.

Die gewichtbasierten Raumgeschwindigkeiten (Reaktandmasse pro Katalysatormasse pro Zeit; weight hourly space velocity (WHSV)) befinden sich beim erfindungsgemäßen Verfahren zur Oligomerisierung vorzugsweise im Bereich zwischen 1 g Reaktand pro g Katalysator und pro h (= $1\ h^{-1}$) und $190\ h^{-1}$, vorzugsweise zwischen $2\ h^{-1}$ und $35\ h^{-1}$, besonders bevorzugt zwischen $3\ h^{-1}$ und $25\ h^{-1}$.

Der erfindungsgemäß eingesetzte Oligomerisierungskatalysator umfasst mindestens Nickeloxid und ein amorphes Alumosilikat als Supportmaterial. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist. Das amorphe Silica-Alumina-Supportmaterial ist kein kristallines Material, beispielsweise kein zeolithisches Material.

Der Nickel-enthaltende Alumosilikatkatalysator, der im erfindungsgemäßen Verfahren eingesetzt wird, ist mesoporös, weist also mindestens Mesoporen auf. Der mittlere Porendurchmesser des eingesetzten Alumosilikatkatalysators beträgt vorzugsweise mindestens 0,7 nm. Der mittlere Porendurchmesser kann mittels Quecksilber-Porosimetrie gemäß DIN 66133 (Stand: 1993-06) bestimmt werden.

Der erfindungsgemäße Nickel-enthaltende Alumosilikatkatalysator umfasst Nickel in einer Menge von 0,1 bis 51 Gew.-%, vorzugsweise 1 bis 42 Gew.-%, besonders bevorzugt 5 bis 33 Gew.-% bezogen auf die Gesamtzusammensetzung des mesoporösen Nickel-enthaltenden Alumosilikatkatalysators. In einer besonders bevorzugten Ausführungsform der vorliegenden Erfindung ist der Oligomerisierungskatalysator im Wesentlichen frei von Titandioxid und/oder Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumdioxid in seiner Gesamtzusammensetzung.

Erfindungsgemäß kann der Nickel-enthaltende Alumosilikatkatalysator eine spezifische Oberfläche (berechnet nach BET) von 150 bis 700 $m^2$/g, vorzugsweise 190 bis 600 $m^2$/g, besonders bevorzugt von 220 bis 550 $m^2$/g aufweisen. Die BET-Oberfläche wird mittels Stickstoff-Physisorption gemäß DIN-ISO 9277 (Stand: 2014-01) gemessen.

In weiterhin bevorzugten Ausführungsform weist der Nickel-enthaltende Alumosilikatkatalysator ein Silicium/Aluminium-Verhältnis (Si/Al) von 1 bis 100, vorzugsweise 2 bis 80, besonders bevorzugt 3 bis 50 auf.

Als Reaktor, der für die Durchführung des erfindungsgemäßen Verfahrens verwendet werden kann, eignen sich dem Fachmann bekannte Reaktoren, in denen eine Oligomerisierung kontinuierlich oder diskontinuierlich durchgeführt werde kann. In einer bevorzugten Ausführungsform wird zur Durchführung des erfindungsgemäßen Oligomerisierungsverfahrens ein Festbettreaktor oder ein Slurryreaktor im kontinuierlichen oder diskontinuierlichen Betrieb verwendet. Das Verfahren wird insbesondere heterogenkatalytisch durchgeführt.

In einer bevorzugten Ausführungsform beträgt der Grad der Dimerisierung (auch "prozentuale Selektivität bezogen auf die Dimerisierung" genannt) bei dem aus der Oligomerisierung erhaltenen Produkt bzw. Produktstrom bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel:

$$\frac{\text{(einfach verzweigte Dimere (Gew.-\%)} + 2 \times \text{zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein $C_9$-Alkoholgemisch. Das $C_9$-Säuregemisch kann zur Herstellung von Schmiermitteln oder Sikkative verwendet werden. Das $C_9$-Alkoholgemisch ist Vorstufe für die Herstellung von Weichmachern, insbesondere von Di-Nonyl-phthalaten oder DINCH.

Beispiel:

Katalysatorsynthese:

Als Katalysator wurde ein amorphes, acides und mesoporöses Alumosilicat in Form von Granulaten mit einem mittleren Partikeldurchmesser von 1 bis 2 mm, einem mittleren Porendurchmesser von 11 nm (bestimmt mittels mittlere Porendurchmesser und Quecksilber-Porosimetrie) und einem Porenvolumen von 1 g/l verwendet. Dieses Alumosilicat (ohne Nickel) dient zur Bestimmung der Produktverteilung der säurekatalysierten Umsetzung von n-Butenen, sowie zur Kalibrierung für die erfindungsgemäßen Bestimmungsverfahren.

Das Alumosilicat wurde weiterhin mit einer wässrigen $Ni(NO_3)_2$-Lösung imprägniert um Nickel einzubringen. Für die Imprägnierung wurde genau so viel Volumen an Lösung verwendet, dass das Porenvolumen gerade eben gefüllt ist. Diese Methode ist dem Fachmann als incipient wetness Imprägnierung bekannt. Die Konzentration an Nickel in der Lösung wurde so angepasst, dass durch die incipient wetness Imprägnierung Alumosilicat-Katalysatoren mit einem Nickelgehalt von 1, 6 und 14 wt.-% erhalten werden. Diese Katalysatoren wurden anschließend bei 550 °C für 10 h in einem Stickstoffstrom calciniert.

Produktverteilung bei den hergestellten Katalysatoren:

Die Oligomerisierung von n-Butenen wurde kontinuierlich in einem Rohrreaktor mit einer Belastung (WHSV) von 7,5 g Olefinen pro Stunde pro Gramm Katalysator durchgeführt. Die Reaktion wurde bei 100 °C und einen Druck von 30 bar durchgeführt. Dadurch konnte sichergestellt werden, dass die Edukte und Produkte in flüssiger Phase vorliegen. Der Austrag der Reaktoren wurde mittels Gaschramotographie analysiert und der Anteil einzelner Octen-Isomere am Produktspektrum bestimmt. Die Anteile an n-Octen (n-O), 3,4-Dimethylhexen (3,4-DMH) und 4,4-Dimethylhexen (4,4-DMH) sind in Tabelle 1 zusammengefasst.

Tabelle 1: Anteile von n-Octen (n-O), 3,4-Dimethylhexen (3,4-DMH) und 4,4-Dimethylhexen (4,4-DMH) am Produktspektrum sowie das Verhältnis von 4,4-DMH zu 3,4-DMH.

| Alumosilicat | n-O | 3,4-DMH | 4,4-DMH | Verhältnis 4,4-DMH zu 3,4-DMH |
|---|---|---|---|---|
| ohne Ni | 0,0 | 26,3 | 1,7 | 0,06 |
| 1 wt.-% Ni | 1,8 | 13,0 | 0,3 | 0,02 |
| 6 wt.-% Ni | 1,9 | 9,6 | 0,1 | 0,01 |
| 14 wt.-% Ni | 2,9 | 5,1 | 0 | 0 |

Das Ziel des vorliegenden Verfahrens ist die Bildung von möglichst linearen Octen-Iosmeren. Der Katalysator mit 14 wt.-% Nickel zeigt die höchste gebildete Menge an n-Octen und somit auch die höchste Selektivität zum linearen Dimer. Mit sinkenden Nickelanteil wird n-Buten vermehrt über einen säurekatalysierten Mechanismus umgesetzt, wodurch der Anteil verzweigter Produkte erhöht wird. Dies äußert sich auch im Verhältnis von 4,4-DMH zu 3,4-DMH. Je höher der Anteil an Säurekatalyse ist, desto größer ist das eben erwähnte Verhältnis. Ein Verhältnis kleiner 0,05 charakterisiert hierbei das Eintreten von koordinativer Katalyse und bei einem Verhältnis kleiner 0,01 wird der höchste Anteil an gewünschtem n-Octen gefunden.

**Patentansprüche**

1. Verfahren zur Oligomerisierung von n-Butenen unter Verwendung eines mesoporösen, Nickel-enthaltenden Alumosilikatkatalysators, der Nickel, berechnet als Nickeloxid NiO, in einer Menge von 0,1 bis 51 Gew.-%, enthält und ein amorphes Alumosilikat umfasst über den ein Eduktstrom, der die n-Butene enthält, unter Bildung eines Produktgemisches geleitet wird, **dadurch gekennzeichnet, dass** das Verhältnis von der Menge des gebildeten 4,4-Dimethylhexens zur Menge des gebildeten 3,4-Dimethylhexens in dem Produktgemisch überwacht und bei Überschreiten eines Grenzwerts für das Verhältnis (Menge 4,4-Dimethylhexen / Menge 3,4-Dimethylhexen) der Katalysator ausgetauscht wird,
wobei der Grenzwert für das Verhältnis (Menge 4,4-Dimethylhexen / Menge 3,4-Dimethylhexen) maximal 0,05 beträgt.

2. Verfahren nach Anspruch 1, wobei das Verfahren zur Oligomerisierung bei einer Temperatur im Bereich von 50 °C bis 200 °C, vorzugsweise von 60 °C bis 130 °C durchgeführt wird.

3. Verfahren nach Anspruch 1 oder 2, wobei das Verfahren zur Oligomerisierung bei einem Druck im Bereich von 10 bar bis 70 bar, vorzugsweise von 15 bar bis 42 bar durchgeführt wird.

4. Verfahren nach einem der Ansprüche 1 bis 3, wobei der beim Verfahren zur Oligomerisierung eingesetzte mesoporöse Nickel-enthaltende Alumosilikatkatalysator Nickel, berechnet als Nickeloxid NiO, in einer Menge von 1 bis 42 Gew.-%, vorzugsweise 5 bis 33 Gew.-% bezogen auf die Gesamtzusammensetzung des mesoporösen Nickel-enthaltenden Alumosilikatkatalysators enthält.

5. Verfahren nach einem der Ansprüche 1 bis 4, wobei der beim Verfahren zur Oligomerisierung eingesetzte mesoporöse Nickel-enthaltende Alumosilikatkatalysator ein Si/Al-Verhältnis von 1 bis 100, vorzugsweise 2 bis 80, besonders bevorzugt von 3 bis 50 aufweist.

6. Verfahren nach einem der Ansprüche 1 bis 5, wobei der mesoporöse Nickel-enthaltende Alumosilikatkatalysator kein Titandioxid und/oder kein Zirkoniumdioxid enthält.

**Claims**

1. Process for oligomerization of n-butenes using a mesoporous, nickel-containing aluminosilicate catalyst, which contains nickel, calculated as nickel oxide NiO, in an amount of 0.1% to 51% by weight and comprises an amorphous aluminosilicate, over which a reactant stream containing the n-butenes is passed to form a product mixture, **characterized in that** the ratio of the amount of the formed 4,4-dimethylhexene to the amount of the formed 3,4-dimethylhexene in the product mixture is monitored and the catalyst is replaced upon exceedance of a threshold value for the ratio (amount of 4,4-dimethylhexene / amount of 3,4-dimethylhexene),
wherein the threshold value for the ratio (amount of 4,4-dimethylhexene / amount of 3,4-dimethylhexene) is not more than 0.05.

2. Process according to Claim 1, wherein the process for oligomerization is performed at a temperature in the range from 50°C to 200°C, preferably from 60°C to 130°C.

3. Process according to Claim 1 or 2, wherein the process for oligomerization is performed at a pressure in the range from 10 bar to 70 bar, preferably from 15 bar to 42 bar.

4. Process according to any of Claims 1 to 3, wherein the mesoporous nickel-containing aluminosilicate catalyst employed in the process for oligomerization contains nickel, calculated as nickel oxide NiO, in an amount of 1% to 42% by weight, preferably 5% to 33% by weight, based on the total composition of the mesoporous nickel-containing aluminosilicate catalyst.

5. Process according to any of Claims 1 to 4, wherein the mesoporous nickel-containing aluminosilicate catalyst employed in the process for oligomerization has an Si/Al ratio of 1 to 100, preferably 2 to 80, particularly preferably of 3 to 50.

6. Process according to any of Claims 1 to 5, wherein the mesoporous nickel-containing aluminosilicate catalyst contains no titanium dioxide and/or no zirconium dioxide.

**Revendications**

1. Procédé pour l'oligomérisation de n-butènes avec utilisation d'un catalyseur aluminosilicate mésoporeux contenant du nickel, qui contient du nickel, calculé en tant qu'oxyde de nickel NiO, en une quantité de 0,1 à 51 % en poids, et comprend un aluminosilicate amorphe, sur lequel est envoyé un courant de produit de départ, qui contient des n-butènes, avec formation d'un mélange de produits, **caractérisé en ce qu'**on surveille dans le mélange de produits le rapport de la quantité du 4,4-diméthylhexène formé à la quantité du 3,4-diméthylhexène formé et lorsqu'est excédée une valeur limite du rapport (quantité de 4,4-diméthylhexène / quantité de 3,4-diméthylhexène) on remplace le catalyseur,
la valeur limite du rapport (quantité de 4,4-diméthylhexène / quantité de 3,4-diméthylhexène) étant au maximum 0,05.

2.  Procédé selon la revendication 1, dans lequel le procédé pour l'oligomérisation est effectué à une température dans la plage de 50 °C à 200 °C, de préférence de 60 °C à 130 °C.

3.  Procédé selon la revendication 1 ou 2, dans lequel le procédé pour l'oligomérisation est effectué sous une pression dans la plage de 10 bars à 70 bars, de préférence de 15 bars à 42 bars.

4.  Procédé selon l'une quelconque des revendications 1 à 3, dans lequel le catalyseur aluminosilicate mésoporeux contenant du nickel, utilisé dans le procédé pour l'oligomérisation, contient du nickel, calculé en tant qu'oxyde de nickel NiO, en une quantité de 1 à 42 % en poids, de préférence 5 à 33 % en poids, par rapport à la composition totale du catalyseur aluminosilicate mésoporeux contenant du nickel.

5.  Procédé selon l'une quelconque des revendications 1 à 4, dans lequel le catalyseur aluminosilicate mésoporeux contenant du nickel, utilisé dans le procédé pour l'oligomérisation, présente un rapport Si/Al de 1 à 100, de préférence 2 à 80, de façon particulièrement préférée de 3 à 50.

6.  Procédé selon l'une quelconque des revendications 1 à 5, dans lequel le catalyseur aluminosilicate mésoporeux contenant du nickel ne contient pas de dioxyde de titane et/ou pas de dioxyde de zirconium.

**IN DER BESCHREIBUNG AUFGEFÜHRTE DOKUMENTE**

**In der Beschreibung aufgeführte Patentdokumente**

- WO 9514647 A1

- WO 2006111415 A1

**In der Beschreibung aufgeführte Nicht-Patentliteratur**

- **HENTSCHEL.** Untersuchungen an Nickeloxidmischkatalysatoren. *Chemie Technik,* 1982, vol. 34 (6), 313-316